(19) 

Europäisches Patentamt
European Patent Office
Office européen des brevets

(11) **EP 4 541 282 A1**

(12) **EUROPEAN PATENT APPLICATION**
published in accordance with Art. 153(4) EPC

(43) Date of publication:
**23.04.2025 Bulletin 2025/17**

(21) Application number: **23855184.0**

(22) Date of filing: **18.08.2023**

(51) International Patent Classification (IPC):
**A61B 5/346** (2021.01)     **A61B 5/00** (2006.01)

(52) Cooperative Patent Classification (CPC):
**A61B 5/00; A61B 5/346**

(86) International application number:
**PCT/KR2023/012267**

(87) International publication number:
**WO 2024/039218 (22.02.2024 Gazette 2024/08)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **19.08.2022  KR 20220103921**
**17.08.2023  KR 20230107866**

(71) Applicant: **Medical AI Co., Ltd.**
**Seoul 06180 (KR)**

(72) Inventors:
• **KWON, Joonmyoung**
**Seoul 06180 (KR)**
• **LEE, Byeongtak**
**Seoul 06180 (KR)**

(74) Representative: **Patentanwaltskanzlei**
**Matschnig & Forsthuber OG**
**Biberstraße 22**
**Postfach 36**
**1010 Wien (AT)**

(54) **METHOD, COMPUTER PROGRAM, AND APPARATUS FOR AUGMENTING BIO-SIGNAL DATA**

(57)     According to one embodiment of the present disclosure for achieving the above-described object, there is disclosed a method of augmenting bio-signal data, which is performed by a computing device. The method includes: for a plurality of augmentation algorithms for augmenting bio-signal data by converting the form or rhythm of each bio-signal, generating a target algorithm based on an optimal augmentation intensity determined for each of the augmentation algorithms or by combining one or more of the plurality of augmentation algorithms; and generating augmented data by augmenting basic data including a bio-signal according to the target algorithm.

FIG. 2

EP 4 541 282 A1

## Description

### Technical Field

The present disclosure relates to technology for augmenting data, and more particularly, to a method, computer program, and apparatus for augmenting bio-signal data.

### Background Art

The amount of training data is one of the factors that highly affect the performance of a model. The reason for this is that when data is insufficient, the features of a dataset are not reflected desirably or the risk of underfitting or overfitting increases. Furthermore, when the balance in the amount of data is not established between labels, there is a risk that a model may be trained to be biased toward a label having a larger amount of data.

Accordingly, there is used a method of supplementing the insufficient amount of data or establishing a balance in the amount of data between labels by increasing the amount of data through a data augmentation technique as desired.

In particular, in the medical field, it is difficult to collect data for the training of a model, so that there are frequent cases where data is insufficient or there is an imbalance between labels. Accordingly, the medical field is a field in which data augmentation may be used more efficiently.

Meanwhile, in the case of data including bio-signals, it is difficult to intuitively determine whether the data generated through an augmentation technique has the same label as existing data. Accordingly, it is necessary to clearly determine the level of augmentation to be performed using existing data. Furthermore, an augmentation technique that takes into consideration various features of bio-signals needs to be performed depending on the purpose for which augmented data is to be used. When data that is not suitable for the purpose and is only large in amount is used, there is a risk that it will interfere with the training of a model. Therefore, it is required to augment data according to an appropriate augmentation technique by taking into consideration the characteristics of a model that uses bio-signal data as training data.

### Disclosure

### Technical Problem

The present disclosure has been conceived in response to the above-described background technology, and is directed to a method, computer program, and apparatus for augmenting bio-signal data that adopt an augmentation algorithm for augmenting data by taking into consideration the performance of a model for predicting a disease using bio-signal data.

However, the objects to be accomplished by the present disclosure are not limited to the object mentioned above, and other objects not mentioned may be clearly understood based on the following description.

### Technical Solution

According to one embodiment of the present disclosure for achieving the above-described object, there is disclosed a method of augmenting bio-signal data. The method includes: for a plurality of augmentation algorithms for augmenting bio-signal data by converting the form or rhythm of each bio-signal, generating a target algorithm based on an optimal augmentation intensity determined for each of the augmentation algorithms or by combining one or more of the plurality of augmentation algorithms; and generating augmented data by augmenting basic data including a bio-signal according to the target algorithm.

Alternatively, generating the target algorithm may include: for each of the augmentation algorithms, generating first experimental data by augmenting bio-signal data based on different augmentation intensities; outputting a performance score of an artificial intelligence model for each augmentation intensity by inputting the first experimental data to the artificial intelligence model; and determining an optimal augmentation intensity among augmentation intensities for each of the augmentation algorithms based on the performance score for each augmentation intensity; wherein the artificial intelligence model is trained to predict a disease or bio-information based on a biosignal.

Alternatively, the augmentation intensity may be calculated based on a signal-to-noise ratio (SNR) between the bio-signal data and the first experimental data.

Alternatively, the optimal augmentation intensity for each of the augmentation algorithms may be an augmentation intensity corresponding to the first experimental data having a highest performance score of the artificial intelligence model.

Alternatively, generating the target algorithm may include determining an augmentation algorithm whose performance score exceeds a reference value to be the target algorithm based on the performance score generated as a result of reflecting therein the optimal augmentation intensity for each of the augmentation algorithms.

Alternatively, generating the target algorithm may include: combining n (n is a natural number equal to or larger than 2) augmentation algorithms among the plurality of augmentation algorithms; outputting a performance score of an artificial intelligence model by inputting second experimental data, generated according to the combined augmentation algorithms, to the artificial intelligence model; and generating the target algorithm based on the performance score; wherein the artificial intelligence model is trained to predict a disease or bio-information based on a bio-signal.

Alternatively, the performance score may vary depending on the type of disease or bio-information predicted by the artificial intelligence model.

Alternatively, the plurality of augmentation algorithms may include: a first augmentation algorithm for preserving the form of a bio-signal while converting the rhythm of the bio-signal; a second augmentation algorithm for converting the form of a bio-signal while preserving the rhythm of the bio-signal; a third augmentation algorithm for converting both the rhythm and form of a bio-signal; and a fourth augmentation algorithm for adding the noise generated by a process of measuring a bio-signal.

Alternatively, the bio-signal may include an electrocardiogram (ECG) signal.

Alternatively, the artificial intelligence model may include an artificial neural network model trained to predict arrhythmia based on an electrocardiogram signal.

According to one embodiment of the present disclosure for achieving the above-described object, there is disclosed a computer program stored in a computer-readable storage medium. The computer program performs operations for augmenting bio-signal data when executed on one or more processors. The operations include operations of: for a plurality of augmentation algorithms for augmenting bio-signal data by converting the form or rhythm of each bio-signal, generating a target algorithm based on an optimal augmentation intensity determined for each of the augmentation algorithms or by combining one or more of the plurality of augmentation algorithms; and generating augmented data by augmenting basic data including a bio-signal according to the target algorithm.

According to one embodiment of the present disclosure for achieving the above-described object, there is disclosed a computing device for augmenting bio-signal data. The computing device includes: a processor including at least one core; memory including program codes executable on the processor; and a network unit for obtaining basic data including a bio-signal. The processor, for a plurality of augmentation algorithms for augmenting bio-signal data by converting the form or rhythm of each bio-signal, generates a target algorithm based on an optimal augmentation intensity determined for each of the augmentation algorithms or by combining one or more of the plurality of augmentation algorithms, and generates augmented data by augmenting basic data including a bio-signal according to the target algorithm.

**Advantageous Effects**

According to the present disclosure, by taking into consideration the feature of medical data in which it is difficult to obtain a large amount of data, data may be augmented while preserving label information, thereby reducing the time and cost for obtaining a large amount of medical data.

In addition, by determining the optimal augmentation intensity and optimal augmentation algorithm combination having high disease prediction performance according to the type of disease and augmenting bio-signal data using this rather than by simply randomly extracting and combining a plurality of augmentation algorithms, data may be augmented to meet the purpose of disease prediction.

**Description of Drawings**

FIG. 1 is a block diagram of a computing device according to one embodiment of the present disclosure;
FIG. 2 is an exemplary diagram showing the configuration of a computing device for augmenting bio-signal data according to one embodiment of the present disclosure;
FIG. 3 is an exemplary diagram showing the relationship between a computing device for augmenting bio-signal data according to one embodiment of the present disclosure and an artificial intelligence model;
FIG. 4 is an exemplary diagram showing the performance scores of an artificial intelligence model according to combinations of augmentation algorithms according to one embodiment of the present disclosure;
FIGS. 5 and 6 are exemplary diagrams showing the performance scores of an artificial intelligence model based on the augmentation intensity according to one embodiment of the present disclosure; and
FIG. 7 is a flowchart showing a method by which a computing device augments bio-signal data according to one embodiment of the present disclosure.

**Mode for Invention**

Embodiments of the present disclosure will be described in detail below with reference to the accompanying drawings so that those having ordinary skill in the art of the present disclosure (hereinafter referred to as those skilled in the art) can easily implement the present disclosure. The embodiments presented in the present disclosure are provided to enable those skilled in the art to use or practice the content of the present disclosure. Accordingly, various modifications to embodiments of the present disclosure will be apparent to those skilled in the art. That is, the present disclosure may be implemented in various different forms and is not limited to the following embodiments.

The same or similar reference numerals denote the same or similar components throughout the specification of the present disclosure. Additionally, in order to clearly describe the present disclosure, reference numerals for parts that are not related to the description of the present disclosure may be omitted in the drawings.

The term "or" used herein is intended not to mean an exclusive "or" but to mean an inclusive "or." That is, unless otherwise specified herein or the meaning is not clear from the context, the clause "X uses A or B" should be understood to mean one of the natural inclusive substitutions. For example, unless otherwise specified herein or the meaning is not clear from the context, the clause "X uses A or B" may be interpreted as any one of a case where X uses A, a case where X uses B, and a case where X uses both A and B.

The term "and/or" used herein should be understood to refer to and include all possible combinations of one or more of listed related concepts.

The terms "include" and/or "including" used herein should be understood to mean that specific features and/or components are present. However, the terms "include" and/or "including" should be understood as not excluding the presence or addition of one or more other features, one or more other components, and/or combinations thereof.

Unless otherwise specified herein or unless the context clearly indicates a singular form, the singular form should generally be construed to include "one or more."

The term "N-th (N is a natural number)" used herein may be understood as an expression used to distinguish the components of the present disclosure according to a predetermined criterion such as a functional perspective, a structural perspective, or the convenience of description. For example, in the present disclosure, components performing different functional roles may be distinguished as a first component or a second component. However, components that are substantially the same within the technical spirit of the present disclosure but should be distinguished for the convenience of description may also be distinguished as a first component or a second component.

The term "obtaining" used herein may be understood to mean not only receiving data over a wired/wireless communication network connecting with an external device or a system, but also generating data in an on-device form.

Meanwhile, the term "module" or "unit" used herein may be understood as a term referring to an independent functional unit processing computing resources, such as a computer-related entity, firmware, software or part thereof, hardware or part thereof, or a combination of software and hardware. In this case, the "module" or "unit" may be a unit composed of a single component, or may be a unit expressed as a combination or set of multiple components. For example, in the narrow sense, the term "module" or "unit" may refer to a hardware component or set of components of a computing device, an application program performing a specific function of software, a procedure implemented through the execution of software, a set of instructions for the execution of a program, or the like. Additionally, in the broad sense, the term "module" or "unit" may refer to a computing device itself constituting part of a system, an application running on the computing device, or the like. However, the above-described concepts are only examples, and the concept of "module" or "unit" may be defined in various manners within a range understandable to those skilled in the art based on the content of the present disclosure.

The term "model" used herein may be understood as a system implemented using mathematical concepts and language to solve a specific problem, a set of software units intended to solve a specific problem, or an abstract model for a process intended to solve a specific problem. For example, a neural network "model" may refer to an overall system implemented as a neural network that is provided with problem-solving capabilities through training. In this case, the neural network may be provided with problem-solving capabilities by optimizing parameters connecting nodes or neurons through training. The neural network "model" may include a single neural network, or a neural network set in which multiple neural networks are combined together.

The foregoing descriptions of the terms are intended to help to understand the present disclosure. Accordingly, it should be noted that unless the above-described terms are explicitly described as limiting the content of the present disclosure, they are not used in the sense of limiting the technical spirit of the present disclosure.

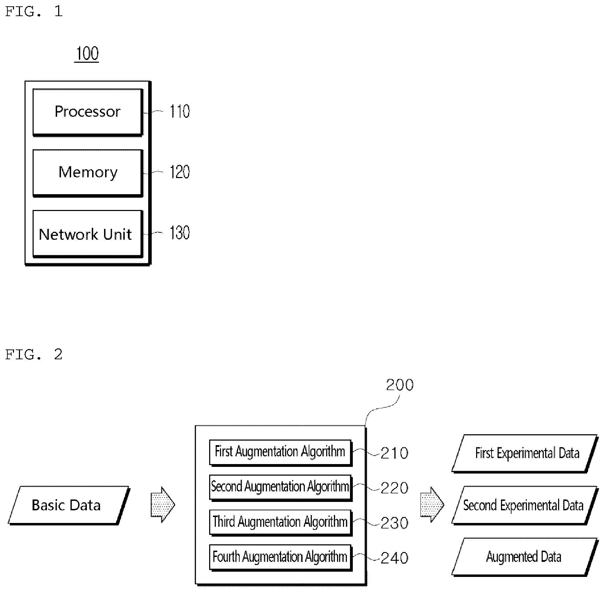

FIG. 1 is a block diagram of a computing device according to one embodiment of the present disclosure.

The computing device 100 according to the one embodiment of the present disclosure may be a hardware device or a part of a hardware device that performs the comprehensive processing and operation of data, or may be a software-based computing environment that is connected to a communication network. For example, the computing device 100 may be a server that is a principal agent performing an intensive data processing function and sharing resources, or may be a client that shares resources through interaction with a server or a specific terminal. Furthermore, the computing device 100 may be a cloud system in which pluralities of servers and clients comprehensively process data while interacting with each other. Furthermore, the computing device 100 may be a component of a medical robot. Since the above-described description is only one example related to the type of the computing device 100, the type of the computing device 100 may be configured in various manners within a range understandable to those skilled in the art based on the content of the present disclosure.

Referring to FIG. 1, the computing device 100 according to one embodiment of the present disclosure may include a processor 110, memory 120, and a network unit 130. However, FIG. 1 shows only an example, and the computing device 100 may include other components for implementing a computing environment. Furthermore, only some of the components disclosed above may be included in the computing device 100.

The processor 110 according to one embodiment of the present disclosure may be understood as a constituent unit including hardware and/or software for performing computing operation. For example, the processor 110 may read a computer program and perform data processing. The processor 110 may process operation processes such as the processing of input data for machine learning, the extraction of features for machine learning, and the computation of errors based on backpropagation. The processor 110 for performing such data processing may include a central processing unit (CPU), a general purpose graphics processing unit (GPGPU), a tensor processing unit (TPU), an application specific integrated circuit (ASIC), or a field programmable gate array (FPGA). Since the types of processor 110 described above are only examples, the type of processor 110 may be configured in various manners within a range understandable to those skilled in the art based on the content of the present disclosure.

The processor 110 according to the present disclosure may adopt or generate a target algorithm among a plurality of augmentation algorithms based on the optimal augmentation intensity determined for each augmentation algorithm in order to augment bio-signal data. Alternatively, the processor 110 may generate the target algorithm by combining one or more of the plurality of augmentation algorithms. The processor 110 may generate augmented data by augmenting basic data including a bio-signal according to the target algorithm.

In this case, the augmented data may be used as training data intended to train an artificial intelligence model that predicts diseases, classifies diseases, determines diseases, or predicts bio-information by using bio-signals. The artificial intelligence model may predict a disease using the augmented data and output a performance score for the prediction of the disease. Alternatively, the artificial intelligence model may predict bio-information such as heart rate and blood pressure using the augmented data and output a performance score indicating the accuracy of the prediction. The performance score may vary depending on training data, i.e., augmented data.

The artificial intelligence model may perform learning based on supervised learning by using training data including samples generated based on bio-signals and labels corresponding to the samples. For example, the samples input to the artificial intelligence model may be electrocardiogram data, and the labels may be cardiovascular diseases. The health state prediction model may include at least one neural network. The neural network may include, but not limited to, at least one of neural networks such as a deep neural network (DNN), a recurrent neural network (RNN), a bidirectional recurrent deep neural network (BRDNN), a multilayer perceptron (MLP), a convolutional neural network (CNN), and a transformer.

Accordingly, the purpose of the processor 110 may be to augment the basic data so that the performance of the artificial intelligence model is improved, that is, so that the performance score of the artificial intelligence model is increased.

For this purpose, in order to generate a target algorithm, the processor 110 may perform an experiment based on a plurality of augmentation algorithms and determine the target algorithm based on the results of the experiment.

For each augmentation algorithm, the processor 110 may generate first experimental data by augmenting bio-signal data, and may output a performance score of the artificial intelligence model for the first experimental data by inputting the first experimental data to the artificial intelligence model.

The processor 110 may augment bio-signal data by applying different augmentation intensities to one augmentation algorithm. Accordingly, a generated result may be first experimental data. That is, for one augmentation algorithm, a performance score may be obtained by inputting data augmented with low augmentation intensity to the artificial intelligence model, and a performance score may be obtained by inputting data augmented with high augmentation intensity to the artificial intelligence model. Among these, an augmentation intensity corresponding to a case where the performance score is high may be determined. The processor 110 may determine this augmentation intensity to be the optimal augmentation intensity of the corresponding augmentation algorithm.

The processor 110 may determine an augmentation algorithm having a high performance score, generated as a result of reflecting the optimal augmentation intensity for each augmentation algorithm, to be the target algorithm based on the results of the experiment using the first experimental data.

In addition, the processor 110 may generate second experimental data by combining and using two or more of the plurality of augmentation algorithms, and may determine the target algorithm based on the performance score of the artificial intelligence model for the second experimental data.

A process of generating the first experimental data will now be specifically described. The processor 110 may use the optimal augmentation intensity in the process of augmenting the basic data using the adopted target algorithm. The augmentation intensity may refer to the ratio of the noise caused by augmentation and the intensity of the bio-signal included in the basic data in the process of augmenting the basic data using each augmentation algorithm. In other words, it may be calculated based on the signal-to-noise ratio (SNR) between the bio-signal data and the corresponding first experimental data. The processor 110 may determine the optimal augmentation intensity for each augmentation algorithm. The optimal augmentation intensity may refer to an augmentation intensity corresponding to a case where the performance score is high.

A process of generating the second experimental data will now be specifically described. The processor 110 may adopt only one or more specific augmentation algorithms capable of improving the performance of the artificial intelligence model in the process of combining one or more specific augmentation algorithms among a plurality of augmentation algorithms. The processor 110 may adopt any two augmentation algorithms among the plurality of augmentation algorithms, may generate second experimental data by combining the adopted two algorithms, and may output a performance score by inputting the second experimental data to the artificial intelligence model. The processor 110 may augment basic data by combining augmentation algorithms having high performance scores based on performance scores.

The processor 110 may use the above-described methods, i.e., the method of adopting an augmentation algorithm based on the optimal augmentation intensity and the method of combining only augmentation algorithms having high performance scores according to the combination, individually or simultaneously. In the case of using the above methods simultaneously, there may be implemented a method in which two augmentation algorithms are adopted and augmentation data is generated according to the optimal augmentation intensity of each of the augmentation algorithms.

According to the present disclosure, by taking into consideration the feature of medical data in which it is difficult to obtain a large amount of data, data may be augmented while preserving label information, thereby reducing the time and cost for obtaining a large amount of medical data.

In addition, by determining the optimal augmentation intensity and optimal augmentation algorithm combination having high disease prediction performance according to the type of disease and augmenting bio-signal data using this rather than by simply randomly extracting and combining a plurality of augmentation algorithms, data may be augmented to meet the purpose of disease prediction.

The memory 120 according to one embodiment of the present disclosure may be understood as a constituent unit including hardware and/or software for storing and managing data that is processed in the computing device 100. That is, the memory 120 may store any type of data generated or determined by the processor 110 and any type of data received by the network unit 130. For example, the memory 120 may include at least one type of storage medium of a flash memory type, hard disk type, multimedia card micro type, and card type memory, random access memory (RAM), static random access memory (SRAM), read-only memory (ROM), electrically erasable programmable read-only memory (EEPROM), programmable read-only memory (PROM), magnetic memory, a magnetic disk, and an optical disk. Furthermore, the memory 120 may include a database system that controls and manages data in a predetermined system. Since the types of memory 120 described above are only examples, the type of memory 120 may be configured in various manners within a range understandable to those skilled in the art based on the content of the present disclosure.

The memory 120 may structure, organize and manage data required for the processor 110 to perform operations, combinations of the data, and program codes executable by the processor 110. For example, the memory 120 may store a plurality of augmentation algorithms, bio-signal data, basic data, first experiment data, second experiment data, and augmented data. Furthermore, the memory 120 may store a performance score based on the first experiment data and a performance score based on the second experiment data for each augmentation algorithm.

The memory 120 may store program codes configured to operate the processor to adopt target algorithms and augment basic data according to the combined target algorithms, and processed data generated as the program codes are executed.

The network unit 130 according to one embodiment of the present disclosure may be understood as a constituent unit that transmits and receives data through any type of known wired/wireless communication system. For example, the network unit 130 may perform data transmission and reception using a wired/wireless communication system such as a local area network (LAN), a wideband code division multiple access (WCDMA) network, a long term evolution (LTE) network, the wireless broadband Internet (WiBro), a 5th generation mobile communication (5G) network, a ultra wide-band wireless communication network, a ZigBee network, a radio frequency (RF) communication network, a wireless LAN, a wireless fidelity network, a near field communication (NFC) network, or a Bluetooth network. Since the above-described communication systems are only examples, the wired/wireless communication system for the data transmission and reception of the network unit 130 may be applied in various manners other than the above-described examples.

The network unit 130 may receive the data required for the processor 110 to perform operations through wired or wireless communication with any system, any client, or the like. Furthermore, the network unit 130 may transmit the data, generated through the operation of the processor 110, through wired or wireless communication with any system, any client, or the like.

For example, the network unit 130 may receive basic data including a bio-signal through communication with a database in a hospital environment, a cloud server that performs tasks such as the standardization of medical data, or the computing device 100. The network unit 130 may transmit first experimental data, second experimental data, augmented data, performance scores of the artificial intelligence model, the intermediate data derived in the operation process of the processor 110, and processed data through communication with the above-described database, server, or computing device 100. Meanwhile, when the first experimental data and the second experimental data are generated outside the computing device 100, the network unit 130 may receive the first experimental data and the second experimental data from the outside.

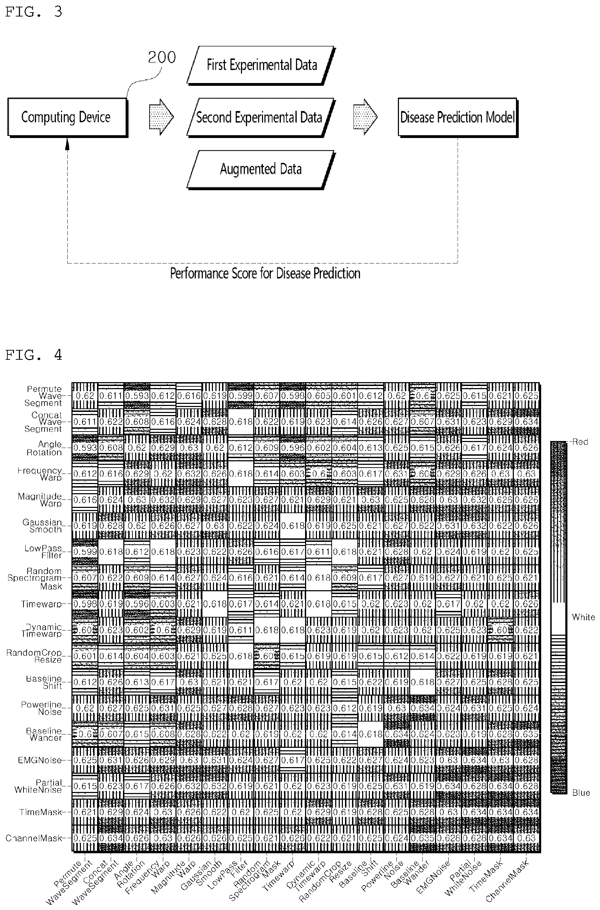

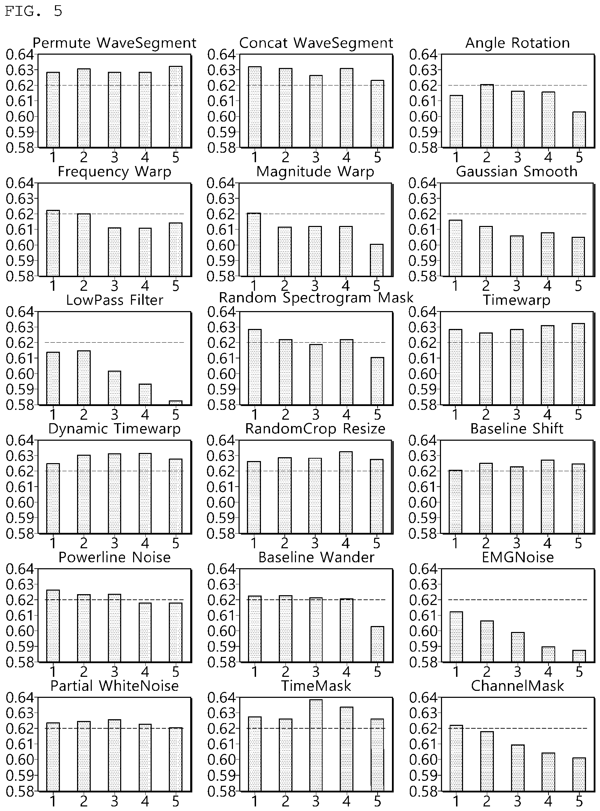

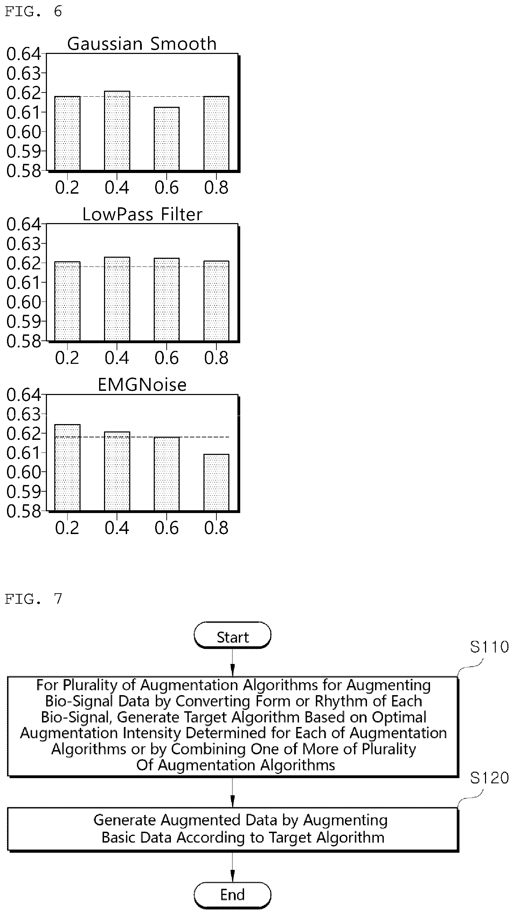

FIG. 2 is an exemplary diagram showing the configuration of a computing device for augmenting bio-signal data according to one embodiment of the present disclosure, FIG. 3 is an exemplary diagram showing the relationship between a computing device for augmenting bio-signal data according to one embodiment of the present disclosure and an artificial intelligence model, FIG. 4 is an exemplary diagram showing the performance scores of an artificial intelligence model according to combinations of augmentation algorithms according to one embodiment of the present disclosure, and FIGS. 5 and 6 are exemplary diagrams showing the performance scores of an artificial intelligence model based on the augmentation intensity according to one embodiment of the present disclosure.

Referring to FIG. 2, the computing device 200 may generate augmented data by augmenting basic data including a bio-signal.

The computing device 200 may generate a target algorithm based on the optimal augmentation intensity determined for each augmentation algorithm or by combining one or more of a plurality of augmentation algorithms, and may generate augmented data using the target algorithm. In this case, the plurality of augmentation algorithms refer to methods of augmenting bio-signal data within the range in which the disease or physical features indicated by a bio-signal are not damaged, and may be a method of augmenting bio-signal data by converting the form or rhythm of a bio-signal.

The plurality of augmentation algorithms may include first to fourth augmentation algorithms 210, 220, 230, and 240. The first augmentation algorithm 210 may be a method of preserving the form of a bio-signal while converting the rhythm of the bio-signal, the second augmentation algorithm 220 may be a method of converting the form of a bio-signal while preserving the rhythm of the bio-signal, the third augmentation algorithm 230 may be a method of converting both the rhythm and form of a bio-signal, and the fourth augmentation algorithm 240 may be a method of adding the noise generated by the process of measuring a bio-signal.

In this case, the rhythm of a bio-signal refers to indicators including the heart rate, the P wave interval, and the R wave interval in the case of an electrocardiogram signal. The form of a bio-signal refers to indicators including the form of an electrocardiogram signal, i.e., the form of the P wave, the form of the QRS wave, and the form of the ST segment in the case of the electrocardiogram signal.

The first augmentation algorithm 210 may include a method of segmenting a bio-signal into a plurality of segments and rearranging the time order of the individual segments, and a method of selecting only a specific segment from among a plurality of segments and duplicating it into its original length. The second augmentation algorithm 220 may include a method of distorting the interval between individual bins by converting a bio-signal into a frequency domain, a method of convolving a bio-signal using a normalized Gaussian function, and a method of filtering a signal having a specific frequency or higher. The third augmentation algorithm 230 may include a method of increasing or decreasing a bio-signal, and a method of segmenting a bio-signal into a plurality of segments and warping them. The fourth augmentation algorithm 240 may include a method of adding the noise caused by disturbance on a power line, and a method of adding muscle contraction high-frequency noise by using Gaussian noise.

Referring to FIG. 3, the augmented data may be input to the artificial intelligence model and used as training data for the artificial intelligence model. That is, the artificial intelligence model may be subjected to supervised learning to predict a disease from a bio-signal based on the features of the bio-signal included in the augmented data. For example, when the bio-signal is an electrocardiogram signal, the artificial intelligence model may be trained to predict arrhythmia based on the electrocardiogram signal. The artificial intelligence model may include mathematical models such as a classification model and a regression model. Alternatively, the artificial intelligence model may be trained to predict future bio-information based on the features of a bio-signal included in the augmented data.

As described above, the computing device 200 may generate a target algorithm based on a plurality of augmentation algorithms by generating the first experimental data or the second experimental data.

First, a process of generating the second experimental data will now be described. In order to adopt one or more of the first to fourth augmentation algorithms 210, 220, 230, and 240, the computing device 200 may input the second experimental data, augmented and generated based on a combination of the first and fourth augmentation algorithms 210, 220, 230, and 240, to the artificial intelligence model, and may use the performance score output from the artificial intelligence model.

Referring to FIGS. 3 and 4 together, the computing device 200 may augment basic data by adopting the combination of the first to fourth augmentation algorithms 210, 220, 230, and 240 that outputs a high performance score. Meanwhile, the performance score may vary depending on the type of disease or bio-information predicted by the artificial intelligence model. In the following description, the computing device 200 is described as selecting two augmentation algorithms, but the number of augmentation algorithms is not limited thereto.

In the graph of FIG. 4, the elements located on the x axis and the y axis represent augmentation algorithms, and the numbers written on the graph represent performance scores. In this case, the numbers marked in red represent improvements in performance, i.e., positive performance scores output as a result of the combination of augmentation algorithms, and the numbers marked in blue represent decreases in performance, i.e., negative performance scores output as a result of the combination of augmentation algorithms.

Accordingly, the computing device 200 may select an augmentation algorithm that outputs a positive performance score based on the graph of FIG. 4. For example, when the computing device 200 selects one augmentation algorithm, the computing device 200 may use the following mathematical equation to determine the remaining augmentation algorithm. In this case, A refers to the graph, i.e., the matrix, shown in FIG. 4.

$$aug_n \sim norm\left(\prod_{i=1}^{n-1} A_{aug_i}, .\right)$$

According to this, when an augmentation algorithm called PermuteWaveSegment is selected first, the computing device 200 may select an augmentation algorithm called ChannelMask or RandomCropResize with a high probability.

Regarding the first experimental data generation process, referring to FIGS. 3, 5, and 6 together, for a plurality of augmentation algorithms including the first through fourth augmentation algorithms 210, 220, 230, and 240, the computing device 200 may generate the first experimental data by augmenting the bio-signal data with different augmentation intensities and may input the first experimental data to the artificial intelligence model. Furthermore, the artificial intelligence model may output a performance score for each augmentation intensity. The computing device 200 may calculate the optimal augmentation intensity for each of the plurality of augmentation algorithms based on the performance scores. In other words, the augmentation intensity having the highest performance score of the artificial intelligence model may be determined to be the optimal augmentation intensity.

Meanwhile, the augmentation intensity may be calculated based on the signal-to-noise ratio (SNR) between the bio-signal data and the first experimental data, and may be based on $SNR^{-1}$ values according to the following equation:

$$SNR^{-1}$$

$$= \frac{rms\left((\text{Biosignal of Biosignal Data before Augmentation}) - (\text{Biosignal of Augmented Biosignal Data})\right)}{rms(\text{Biosignal of Biosignal Data before Augmentation})}$$

For example, the computing device 200 may make $SNR^{-1}$ values, distributed in the range of 0 to 1, correspond to five segments. That is, the size in which $SNR^{-1}$ values correspond to the value range of 0 to 0.2 may correspond to the first segment, and the size in which $SNR^{-1}$ values correspond to the value range of 0.8 to 1 may correspond to the fifth segment.

For example, for an augmentation algorithm called A, the computing device 200 may augment data by adjusting the augmentation intensity to 0, 0.2, 0.4, 0.6, 0.8, and 1.0. A total of six types of first experimental data may be generated according to A. The computing device 200 may output performance scores by inputting the six types of first experimental data to the artificial intelligence model. For example, the performance score may be highest when the augmentation intensity is 0.6. The computing device 200 may determine the optimal augmentation intensity of A to be 0.6.

Referring to FIGS. 5 and 6, each graph is intended for each augmentation algorithm, the x axis represents 5 segments, and the y axis represents the performance scores of the artificial intelligence model. FIG. 6 shows the results of sub-dividing the segments and then deriving performance scores again for the augmentation algorithms, such as EMGNoise, GaussianSmoothing, and LowPassFilter, whose performance scores decrease for all augmentation intensities. As a result, the performance score tends to increase as the augmentation intensity increases, but the performance score tends to decrease again when the augmentation intensity exceeds a specific magnitude. Accordingly, the computing device 200 may determine the optimal augmentation intensity having the highest performance score. In other words, the computing device 200 may determine the optimal ratio between original data before augmentation and augmented data so that the performance of the artificial intelligence model is high, for each augmentation algorithm. Based on this, the computing device 200 may determine the augmentation algorithm whose performance score exceeds a reference value to be the target algorithm based on the performance scores generated as a result of reflecting therein the optimal augmentation intensity for each augmentation algorithm.

Accordingly, the computing device 200 of the present disclosure may adopt a data augmentation technique that exhibits optimal performance in order not to degrade the performance of the artificial intelligence model in which augmented data is utilized. Furthermore, data that is unrelated to performance or that degrades performance is not used for training, so that the artificial intelligence model can be efficiently trained.

FIG. 7 is a flowchart showing a method by which a computing device augments bio-signal data according to one embodiment of the present disclosure.

In the following description, the bio-signal may include an electrocardiogram (ECG) signal, and the artificial intelligence model may include an artificial neural network model trained to predict arrhythmia based on a bio-signal.

Referring to FIG. 7, the computing device 100 or 200 may generate a target algorithm based on the optimal augmentation intensity determined for each augmentation algorithm or by combining one or more of a plurality of augmentation algorithms, for the plurality of augmentation algorithms for augmenting bio-signal data by converting the form or rhythm of each bio-signal in step S110.

In this case, for each of the plurality of augmentation algorithms, the computing device 100 or 200 may generate first experimental data by augmenting bio-signal data based on different augmentation intensities and output the performance score of the artificial intelligence model for each augmentation intensity by inputting the first experimental data to the artificial intelligence model. Furthermore, based on the performance score for each augmentation intensity, the optimal augmentation intensity for each of the augmentation algorithms may be determined.

The augmentation intensity may be calculated based on the signal-to-noise ratio (SNR) between the bio-signal data and the first experimental data, and the above-mentioned value may be used as the augmentation intensity. The augmentation intensity corresponding to the first experimental data having the highest performance score of the artificial intelligence model may be determined to be the optimal augmentation intensity.

The computing device 100 or 200 may determine an augmentation algorithm, whose performance score exceeds a reference value, to be the target algorithm based on the performance score generated as a result of reflecting therein the optimal augmentation intensity for each augmentation algorithm.

In addition, the computing device 100 or 200 may combine n (n is a natural number equal to or larger than 2) augmentation algorithms among a plurality of augmentation algorithms. The computing device 100 or 200 may output the performance score of the artificial intelligence model by inputting the second experimental data, generated according to the combined augmentation algorithms, to the artificial intelligence model, and may generate the target algorithm based on the performance score. The performance score may vary depending on the type of disease predicted by the artificial intelligence model. For example, an augmentation algorithm called Smoothing is an effective augmentation method for predicting a Right Axis Deviation (RAD), but is not effective for predicting a Bundle branch block (BBB). Accordingly, the performance score of Smoothing will be high in the artificial intelligence model for predicting an RAD, but will be low in the artificial intelligence model for predicting a BBB.

In this case, the plurality of augmentation algorithms may include one or more of the first augmentation algorithm 210 for preserving the form of a bio-signal while converting the rhythm of the bio-signal, the second augmentation algorithm 220 for converting the form of a bio-signal while preserving the rhythm of the bio-signal, the third augmentation algorithm 230 for converting both the rhythm and form of a bio-signal, and the fourth augmentation algorithm for adding the noise generated by the process of measuring a bio-signal.

The computing device 100 or 200 may generate augmented data by augmenting basic data including a bio-signal according to the target algorithm in step S120.

The various embodiments of the present disclosure described above may be combined with one or more additional embodiments, and may be changed within the range understandable to those skilled in the art in light of the above detailed description. The embodiments of the present disclosure should be understood as illustrative but not restrictive in all respects. For example, individual components described as unitary may be implemented in a distributed manner, and similarly, the components described as distributed may also be implemented in a combined form. Accordingly, all changes or modifications derived from the meanings and scopes of the claims of the present disclosure and their equivalents should be construed as being included in the scope of the present disclosure.

**Claims**

1. A method of augmenting bio-signal data, the method being performed by a computing device including at least one processor, the method comprising:

   for a plurality of augmentation algorithms for augmenting bio-signal data by converting a form or rhythm of each bio-signal, generating a target algorithm based on an optimal augmentation intensity determined for each of the augmentation algorithms or by combining one or more of the plurality of augmentation algorithms; and
   generating augmented data by augmenting basic data including a bio-signal according to the target algorithm.

2. The method of claim 1, wherein generating the target algorithm comprises:

   for each of the augmentation algorithms,
   generating first experimental data by augmenting bio-signal data based on different augmentation intensities;

outputting a performance score of an artificial intelligence model for each augmentation intensity by inputting the first experimental data to the artificial intelligence model; and

determining an optimal augmentation intensity among augmentation intensities for each of the augmentation algorithms based on the performance score for each augmentation intensity;

wherein the artificial intelligence model is trained to predict a disease or bio-information based on a biosignal.

3. The method of claim 2, wherein the augmentation intensity is calculated based on a signal-to-noise ratio (SNR) between the bio-signal data and the first experimental data.

4. The method of claim 3, wherein the optimal augmentation intensity for each of the augmentation algorithms is an augmentation intensity corresponding to the first experimental data having a highest performance score of the artificial intelligence model.

5. The method of claim 4, wherein generating the target algorithm comprises determining an augmentation algorithm whose performance score exceeds a reference value to be the target algorithm based on the performance score generated as a result of reflecting therein the optimal augmentation intensity for each of the augmentation algorithms.

6. The method of claim 1, wherein generating the target algorithm comprises:

combining n (n is a natural number equal to or larger than 2) augmentation algorithms among the plurality of augmentation algorithms;

outputting a performance score of an artificial intelligence model by inputting second experimental data, generated according to the combined augmentation algorithms, to the artificial intelligence model; and

generating the target algorithm based on the performance score;

wherein the artificial intelligence model is trained to predict a disease or bio-information based on a bio-signal.

7. The method of claim 6, wherein the performance score varies depending on a type of disease or bio-information predicted by the artificial intelligence model.

8. The method of claim 6, wherein the plurality of augmentation algorithms comprise:

a first augmentation algorithm for preserving a form of a bio-signal while converting a rhythm of the bio-signal;

a second augmentation algorithm for converting a form of a bio-signal while preserving a rhythm of the bio-signal;

a third augmentation algorithm for converting both a rhythm and form of a bio-signal; and

a fourth augmentation algorithm for adding noise generated by a process of measuring a bio-signal.

9. The method of claim 2, wherein the bio-signal comprises an electrocardiogram (ECG) signal.

10. The method of claim 9, wherein the artificial intelligence model comprises an artificial neural network model trained to predict arrhythmia based on an electrocardiogram signal.

11. A computer program stored in a computer-readable storage medium, the computer program performing operations for augmenting bio-signal data when executed on one or more processors, wherein the operations comprise operations of:

for a plurality of augmentation algorithms for augmenting bio-signal data by converting a form or rhythm of each bio-signal, generating a target algorithm based on an optimal augmentation intensity determined for each of the augmentation algorithms or by combining one or more of the plurality of augmentation algorithms; and

generating augmented data by augmenting basic data including a bio-signal according to the target algorithm.

12. A computing device for augmenting bio-signal data, the computing device comprising:

a processor including at least one core;

memory including program codes executable on the processor; and

a network unit for obtaining basic data including a bio-signal;

wherein the processor, for a plurality of augmentation algorithms for augmenting bio-signal data by converting a form or rhythm of each bio-signal, generates a target algorithm based on an optimal augmentation intensity determined for each of the augmentation algorithms or by combining one or more of the plurality of augmentation algorithms, and generates augmented data by augmenting basic data including a bio-signal according to the target algorithm.

FIG. 1

<u>100</u>

| | |
|---|---|
| Processor | ~ 110 |
| Memory | ~ 120 |
| Network Unit | ~ 130 |

FIG. 2

200

Basic Data

| | |
|---|---|
| First Augmentation Algorithm | ~210 |
| Second Augmentation Algorithm | ~220 |
| Third Augmentation Algorithm | ~230 |
| Fourth Augmentation Algorithm | ~240 |

First Experimental Data

Second Experimental Data

Augmented Data

FIG. 3

200

First Experimental Data

Computing Device

Second Experimental Data

Disease Prediction Model

Augmented Data

Performance Score for Disease Prediction

FIG. 4

Red

White

Blue

FIG. 5

FIG. 6

FIG. 7

<div align="center">**INTERNATIONAL SEARCH REPORT**</div>

| International application No. |
|---|
| **PCT/KR2023/012267** |

**A. CLASSIFICATION OF SUBJECT MATTER**

**A61B 5/346**(2021.01)i; **A61B 5/00**(2006.01)i

According to International Patent Classification (IPC) or to both national classification and IPC

**B. FIELDS SEARCHED**

Minimum documentation searched (classification system followed by classification symbols)

A61B 5/346(2021.01); A61B 5/00(2006.01); A61B 5/327(2021.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 생체신호 데이터 증강(body signal data augmentation), 복수의 증강 알고리즘 (a plurality of augmentation algorithms), 최적의 증강 강도(optimal augmentation intensity), 성능 점수(performance score), 심전도(electrocardiogram), 부정맥 예측(arrhythmia prediction)

**C. DOCUMENTS CONSIDERED TO BE RELEVANT**

| Category* | Citation of document, with indication, where appropriate, of the relevant passages | Relevant to claim No. |
|---|---|---|
| X <br> Y | RAGHU, Aniruddh et al. Data augmentation for electrocardiograms. Proceedings of Machine Learning Research, Conference on Health, Inference, and Learning. April 2022, vol. 174, pp. 282-310. <br> See abstract; pages 283-287; and figures 2-3. | 1,11-12 <br> 2-10 |
| Y | NONAKA, Naoki et al. Data augmentation for electrocardiogram classification with deep neural network. arXiv: 2009.04398v1. pp. 1-6, 2020. Retrieved from <https://arxiv.org/pdf/2009.04398v1.pdf>. <br> See abstract; pages 2-4; and table 1. | 2-10 |
| A | DO, Edmund et al. Data augmentation for 12-lead ECG beat classification. SN Computer Science. 2022 (online publication date: 19 November 2021), vol. 3, article no. 70, pp. 1-17. <br> See entire document. | 1-12 |
| A | LUO, Yun et al. Data augmentation for enhancing EEG-based emotion recognition with deep generative models. Journal of Neural Engineering. 2020, vol. 17, article no. 056021, pp. 1-17. <br> See entire document. | 1-12 |

☑ Further documents are listed in the continuation of Box C.　　☑ See patent family annex.

| * Special categories of cited documents: | |
|---|---|
| "A" document defining the general state of the art which is not considered to be of particular relevance | "T" later document published after the international filing date or priority date and not in conflict with the application but cited to understand the principle or theory underlying the invention |
| "D" document cited by the applicant in the international application | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **22 November 2023** | **22 November 2023** |

| Name and mailing address of the ISA/KR | Authorized officer |
|---|---|
| **Korean Intellectual Property Office** <br> **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| International application No. |
| :--- |
| **PCT/KR2023/012267** |

**C.   DOCUMENTS CONSIDERED TO BE RELEVANT**

| Category* | Citation of document, with indication, where appropriate, of the relevant passages | Relevant to claim No. |
| :---: | :--- | :---: |
| A | KR 10-2022-0098600 A (BODYFRIEND CO., LTD. et al.) 12 July 2022 (2022-07-12)<br>See entire document. | 1-12 |
| PX | LEE, Byeong Tak et al. Efficient data augmentation policy for electrocardiograms. Proceedings of the 31st ACM International Conference on Information and Knowledge Management (CIKM '22). 17-21 October 2022, pp. 4153-4157.<br>See abstract; pages 4153-4156; and table 1. | 1-12 |

Form PCT/ISA/210 (second sheet) (July 2022)

**INTERNATIONAL SEARCH REPORT**
Information on patent family members

International application No.

**PCT/KR2023/012267**

| Patent document cited in search report | Publication date (day/month/year) | Patent family member(s) | Publication date (day/month/year) |
|---|---|---|---|
| KR  10-2022-0098600  A | 12 July 2022 | None | |

Form PCT/ISA/210 (patent family annex) (July 2022)